# EUROPEAN PATENT APPLICATION

(11) **EP 3 757 555 A1**
(43) Date of publication of application: **30.12.2020**
(21) Application number: 19020406.5
(22) Date of filing: 28.06.2019
(51) Int. Cl.: G01N 22/00, G01N 33/02

(54) **METHODS FOR CALCULATING & PREDICTING THE DEGREE-OF-CRYSTALLIZATION OF A PRODUCT**

(71) Applicant: Aquantis SA, 1015 Lausanne (CH)
(72) Inventor: Vandermeiren, Werner, B-1930 Zaventam (BE); Pandey, Gokarna, B-1200 Brussels (BE); Zhang, Yuchen, B-3640 (BE); Van Eeghem, Vincent, 1004 (CH)

(57) **Abstract**

The present invention relates to the field of industrial freezing applications. It relates to methods for calculating and / predicting the degree-of-crystallization of a product. The method comprises exposing the product under test with electromagnetic radiation with at least one frequency within the range of 0.1 GHz to 1 THz and acquiring the electromagnetic transmission and / or reflection signal. The method furthermore comprises calculating the product insertion loss and the normalised product absorbance. The method comprises calculating the degree-of-crystallization based on the measured normalized product absorbance and the ice-fraction dependent dielectric permittivity of the product under test. The method optionally comprises profiling the degree-of-crystallization of a thermally non-equilibrated product and predicting the degree-of-crystallization the product will achieve when it reaches thermal equilibrium assuming energy conservation.

## Description

### Field of the invention

The present invention relates to the field of freezing and thawing of products, but can be generalized to other crystallization processes. More particularly the present invention relates to methods for calculating and predicting the degree-of-crystallization of a product, further referred to as DOC, based on an electromagnetic transmission measurement. Within the scope of freezing and thawing of food products, DOC can be defined as the relative portion of the ice-fraction with respect to the total freezable water fraction within the product under test.

### Background of the invention

One of the most important methods of preservation includes freezing. Perishable products or products whose properties need to be kept inalterable for long periods of time are usually frozen and kept at low temperatures, typically significantly lower than the melting point of ice, for a desired preservation time. This is normally done with food and agricultural products, organs for transplantation, biological and bioengineering materials, medicines and pharmacological products, and chemical products in general.

Another method of preservation is lyophilization or freeze-drying, which involves freezing the product, lowering pressure, then removing the ice by sublimation. This method is usually applied to food (vegetables, beverages, etc.), pharma (vaccines, blood, etc.) and biotechnological and chemical materials (proteins, enzymes, artificial skin, etc.), and occasionally to ceramic products (e.g. in semiconductor industry) or restoration (in archaeology and recovery of water-damaged documents or goods).

The freezing / drying process causes state changes in the products. These are controlled by measuring variables such as the temperature and pressure. For example, in food industry, the freezing lines are equipped with numerous sensors, such as pressure, humidity, air-flow and temperature sensors, for monitoring and controlling the freezing process in-line. However, these measurements do not provide direct information about the degree-of-crystallization of the product under consideration. This usually gives a large uncertainty about the actual state of the product at the end of the freezing line. This uncertainty is even higher for cryogenically frozen products (fast freezing), where one is interested in the DOC of the product when it reaches a thermal equilibrium. A cryogenically frozen product is characterized by large thermal gradients within the product as a consequence of the freezing speed. Typically, the outside and core of the product can be considered as, respectively, over and under-frozen with respect the final DOC the product will achieve at thermal equilibrium. Hence, the crystallization of the product core will continue during thermal equilibration after the outfeed of the freezing installation. During this fast freezing process a significant amount of heat is removed from the products outer layer (shell), such that after the outfeed of the cryogenic freezing installation this 'cold' present in the products outer layer can 'absorb' the latent heat from the liquid water present in the product core which is required to continue the freezing process. Its important to know the products equilibrated DOC for controlling and optimizing the freezing process parameters. Hence, there exist a need for predicting the equilibrated DOC level based on DOC measurements on non-equilibrated product, just after the freezer installation outfeed.

In ordinary productions, the freezer settings are chosen as a function of environmental conditions, the product and the product throughput with a reasonably big safety margin to ensure that the product is law-regulation compliant. This typically implies a thermally equilibrated product temperature lower or equal to -18 °C. Temperature, however, is not always a good indicator for the products DOC as, during the latent heat phase of the freezing-curve, temperature shows a very low sensitivity with respect to the DOC of the product. In this phase, the energy withdrawn from the product results mostly in crystallization (latent heat of water), and to a much lesser extend in product temperature decrease (heat capacity). Nevertheless, a temperature measurement is still the most common method used today. Generally, the -18 °C threshold validation is verified by inserting a dedicated temperature probe (e.g. a PT100 probe) into the product package or in between filled boxes after palletising. This temperature measurement method is indirect, intrusive, labour-intensive, simplistic and it usually can only provide information of the state of the product surface, or the environment in proximity of the product, which leaves uncertainties about the state of the product-core, as the outside layers might be frozen completely, while the product core is not.

Methods such as producing indentations on the product and visual inspection have similar shortcomings. Additionally, none of these methods take into account the heterogeneity of morphology (e.g. the humidity and gradient of humidity, the ice fraction, etc.) of the products at a macro scale.

Concerning the lyophilisation process, the two drying steps involved can be reasonably well-monitored in-line by various methods. This is not the case for the freezing step due to a lack of a direct and in-line DOC measurement device and method. As freezing is usually the first step in the process, it is considered to be critical. When freezing process is not well executed, the consecutive steps will be negatively influenced.

The present invention allows to optimize different kinds of freezing processes within a wide range of industries in terms of throughput and energy consumption, without product quality deterioration and provides peace of mind.

### Summary of the invention

It is an object of embodiments of the present invention to provide good methods for calculating and / or predicting the degree-of-crystallization of one or more products under test.

It is an advantage of particular embodiments of the present invention to provide a method to translate a through-product electromagnetic transmission signal into the **instantaneous degree-of-crystallization** of the product under test.

It is an advantage of particular embodiments of the present invention to provide a method to translate a through-product electromagnetic transmission signal into the **instantaneous product temperature.**

It is an advantage of particular embodiments of the present invention to provide a method to translate a product **degree-of-crystallization** into a **product temperature.** This method can be based on an analytical expression of the form: $T = {T}_{F} / \left(1-DOC\right)$ where T, T_{F} and DOC are the product temperature, the products freezing point and the products degree-of-crystallization.

It is an advantage of particular embodiments of the present invention to provide a method to translate a **product temperature** into a **product degree-of-crystallization.** This method can be based on an analytical expression of the form: $DOC = 1 - \left({T}_{F}/T\right)$ where T, T_{F} and DOC are the product temperature, the products freezing point and the products degree-of-crystallization.

It is an advantage of particular embodiments of the present invention to provide a method to predict the final thermally **equilibrated degree-of-crystallization** of the product under test, based on through-product electromagnetic transmission signals, measured on that same product prior to reach thermal equilibrium, assuming energy conservation on that product.

It is an advantage of particular embodiments of the present invention to provide a method to predict the final thermally **equilibrated product temperature,** based on through-product electromagnetic transmission signals, measured on that same product prior to reach thermal equilibrium, assuming energy conservation on that product.

It is an advantage of embodiments of the present invention to provide methods for **optimizing industrial freezing processes** in terms of efficiency and product throughput.

It is an advantage of particular embodiments of the present invention to provide methods **to calculate the dielectric properties of the product** under test as a function of the dielectric properties of a non-exhaustive and non-restrictive list of its ingredients and the volumetric fractions of these ingredients:
▪ water
▪ ice
▪ carbohydrates
▪ proteins
▪ fats
▪ etc.

The method can include an electromagnetic mixing formula of the form: ${{ε}_{product}}^{β} = {\sum }_{i = 0}^{n} {f}_{i} {{ε}_{i}}^{β}$ where ε_{product}, εᵢ, fᵢ and β are the dielectric permittivity of the product under test, the dielectric permittivities of the product ingredients, the volumetric fractions of the product ingredients and the electromagnetic mixing coefficient (typically 1/3), respectively.

It is an advantage of particular embodiments of the present invention to provide methods **to calculate the dielectric properties of individual** ingredients of the product under test (water, ice, carbohydrates, proteins, fats, etc.) based on a non-exhaustive and non-restrictive list of physical properties:
▪ the electromagnetic wavelength used,
▪ the product temperature.

It is an advantage of particular embodiments of the present invention to provide methods to calculate **a normalized through-product electromagnetic transmission absorbance** (e.g. expressed in dB/mm) as a function a non-exhaustive and non-restrictive list of physical properties:
▪ the electromagnetic transmission loss (e.g. expressed in dB),
▪ the electromagnetic reflection loss (e.g. expressed in dB)
▪ the electromagnetic scattering loss (e.g. expressed in dB or dB/mm),
▪ the product thickness (e.g. expressed in mm).

It is an advantage of particular embodiments of the present invention to provide methods **to** calculate **the relation** between the normalized through-product electromagnetic **transmission absorbance** and the **degree-of-crystallization** of the product under test

It is an advantage of particular embodiments of the present invention to provide methods to calculate:
▪ **a product averaged** normalized through-product electromagnetic transmission absorbance and/or
▪ **a product averaged** degree-of-crystallization and/or
▪ **a product averaged temperature**
   from **measurements taken at different positions** on the product under test.

It is an advantage of particular embodiments of the present invention to provide methods to calculate:
▪ a normalized through-product electromagnetic transmission absorbance **profile** and/or
▪ a degree-of-crystallization **profile** and/or
▪ the product temperature profile
   from a **line-scan profile measurement** on the product under test.

It is an advantage of particular embodiments of the present invention to provide methods to calculate the thermally **equilibrated degree-of-crystallization and/or** the thermally **equilibrated product temperature** based on:
▪ a normalized through-product electromagnetic transmission absorbance **profile** and/or
▪ a degree-of-crystallization **profile** and/or
▪ the product temperature profile
   from a **line-scan profile measurement** on the product under test.

It is an advantage of particular embodiments of the present invention to provide methods to calculate the averaged thermally **equilibrated degree-of-crystallization** and / or the averaged thermally **equilibrated product temperature** over multiple measured products

It is an advantage of particular embodiments of the present invention to provide methods **to detect the presence of a product under** test based on through-product electromagnetic transmission data and a user selectable detection threshold.

It is an advantage of particular embodiments of the present invention to provide methods **to detect the presence of a product under test** based on a product thickness measurement and a user selectable detection threshold.

It is an advantage of particular embodiments of the present invention to provide methods **to accept or reject measurement data taken on a product under test** based on:
▪ the product interaction time with the electromagnetic waves and / or
▪ the product thickness,
   whereby, the product interaction time and / or the product thickness are compared to a user defined acceptance validity range.

### Brief description of the drawings

In what follows, particular exemplary embodiments are discussed with reference to the figures, nonetheless these embodiments are not limiting. The present invention provides many applicable inventive concepts, which can be embodied in a wide variety of specific contexts. The specific embodiments discussed are merely illustrative of specific ways to implement and use the methods of the invention, and do not limit the scope of the invention. The drawings described are thus only schematic and are not limiting. In the drawings, the size of some of the elements may be exaggerated and not drawn on scale for illustrative purposes. The dimensions and relative dimensions do not correspond to actual reductions to practice of the invention.

It is to be noted that the term 'comprising' should not be interpreted as being restricted to the means listed thereafter; it does not exclude other elements, methods or steps. It is thus to be interpreted as specifying the presence of the stated features, methods, steps or components as referred to, but does not preclude the presence or addition of one or more other features, methods, steps or components, or groups thereof.

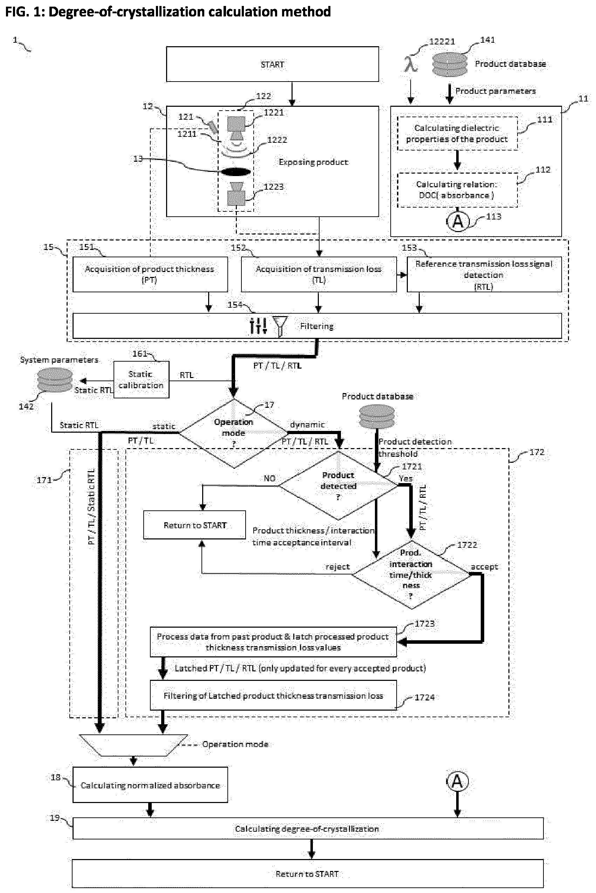

FIG. 1 illustrates a method 1 for calculating the DOC from electromagnetic transmission data for static and/or moving products 13 under test. The method 1 supports the following two operation modes:
▪ static mode-of-operation 171: products 13 are statically place under the sensors aperture
▪ dynamic mode-of-operation 172: products 13 are passing the aperture of the sensor

The method 1, comprising a software module 11 for modelling the dependence between the DOC and the normalized product absorbance for the wavelength 12221 under consideration. These software methods 11 optionally comprises a product dielectric permittivity calculator 111 and a DOC-absorbance relation extractor 112. The result is, dependent on the implementation, a product specific look-up / interpolation table 113 or an analytical expression 113, giving the relation between the DOC and the expected normalized product absorbance, for later use in the DOC calculator software module 19. The methods 11 need to be executed only once when a new product is selected from the product database 141. Hence, once the interpolation table 113 is build, it can be reused for all absorbance measurements made on the selected product from the product database 141.

The method 1, comprising exposing 12 the product 13 with electromagnetic radiation 1222, emitted from an electromagnetic emitter 1221, whereby the transmitted electromagnetic signal is detected by the electromagnetic receiver 1223.

The method 1 furthermore comprises a software module 152 for acquiring and calculating the transmission loss (TL) signal obtained from the electromagnetic receiver hardware 1223. Alternatively, the functionality of this software module 152 could be implemented in hardware on a FPGA.

The method 1 optionally comprises a software module 153 for continuously detecting a reference electromagnetic transmission loss (RTL) signal in the absence of a product under test 13, and keeping this reference transmission loss signal while products 13 are passing the sensor. Having this reference transmission loss signal (measured in the absence of any product 13) available during product 13 measurements allows to calculate the electromagnetic insertion loss of the passing products 13 under test (dynamic mode-of-operation). The software module 153 could be implemented as a leaky peak detector, but is not limited thereto. Alternatively, the functionality of this software module 153 could be implemented in hardware on a FPGA.

The method 1 optionally comprises a software module 151 for acquiring and calculating the product or product layer 13 thickness (PT), obtained from the product thickness detection means 121. This product thickness detection means can be based on one or more laser distance sensors, whereby the laser beam 1211 is pointed to the product under test 13. Note that the product thickness detection means is not limited to laser based technologies. Alternatively, the functionality of this software module 151 could be implemented in hardware on a FPGA.

The method 1 optionally comprises a software module 154 for filtering the input signals streams: PT, TL and RTL. The software module 154 consist of different submodules (filters) which are interconnected to create a filtering recipe. Examples of these submodules are:
▪ moving median filter with software controlled intensity (filter size)
▪ moving averaging filter with software controlled intensity (filter size)
▪ exponentially weighted moving averaging filter with software controlled intensity (parameterized)
▪ others

Alternatively, the functionality of this software module 154 could be implemented in hardware on a FPGA.

The method 1 optionally comprises a software module 161 for on-demand calculating and storing the reference transmission loss signal (measured in the absence of any product 13) to a system parameter database 142. In static mode-of-operation 171 this static reference transmission loss signal is read from the system parameter database 142 to calculated the product insertion loss of the static product 13 under test.

The method 1 optionally comprises a conditional expression 17 to select a signal processing recipe depending on the sensors mode-of-operation.

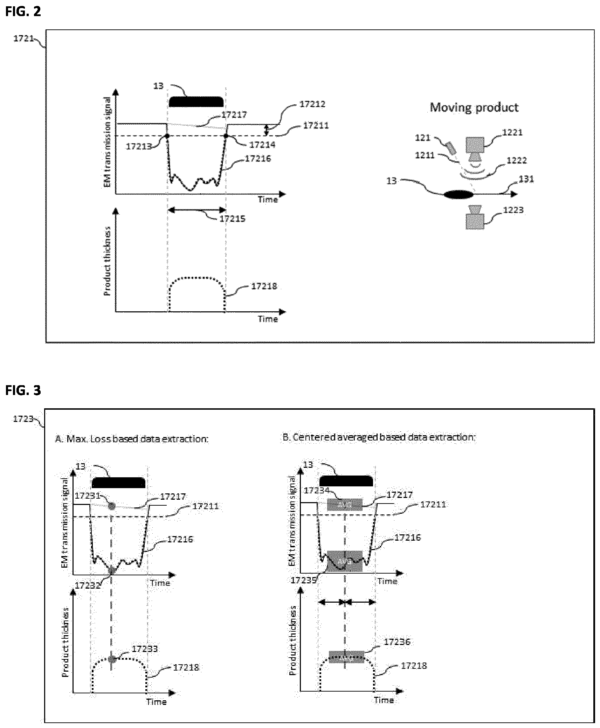

The method 1 optionally comprises a software module 1721 for detecting individual passing products 13 and for registration of temporal evolution of the measurement data (PT, TL and RTL) collected on these products 13. This is illustrated in **FIG. 2****.** The product detection algorithm of the software module 1721 is based on the electromagnetic transmission loss signal (TL). The TL signal is continuously compared to a TLthreshold 17211, whereby the TL threshold is defined as the difference between the RTL signal 17217 and a relative user defined product specific TL threshold offset 17212 (with respect to the RTL level) stored in the product database 141. When abs(TL) > abs( TL threshold ), the presence of a product is detected. When this happened for the first time for a given passing product, the start of that product 17213 is detected and the measurement data storage arrays are cleared. During the product exposure, measurement data profiles (the PT profile 17218, the TL profile 17216 and the RTL profile 17217) are collected and stored in memory. The measurement data registration stops when abs(TL) becomes smaller compared to abs(TL threshold), indicating the end of the product 17214. Alternatively, the product presence detection could be implemented based on the product thickness measurement signal instead of the product 13 induced transmission loss signal.

The method 1 optionally comprises a software module 1722 for detecting whether the measured product 13 can be accepted for further processing. More precisely, software module 1722 will verify based on the registered measurement data profiles if
▪ the product interaction time 17215 is within an acceptance ranges as specified in the product database 141,
▪ the product thickness 17233 is within an acceptance ranges as specified in the product database 141.

In case both conditions are met, the registered measurement data profiles are passed to the product feature extraction module 1723, illustrated in **FIG. 3****.** The product feature extraction module 1723 will extract measurement data around the center of the product 13 and supports different user selectable output types:
▪ Highest measured transmission loss based data extraction: TL 17232, RTL 17231 and PT 17233 values extracted from the registered measurement data profiles (the TL-profile 17216, the RTL-profile 17217 and the PT-profile 17218), where the TL 17232, RTL 17231 and PT 17233 values are taken at the point in the data profiles where the abs(TL) value reaches a maximum, corresponding to a minimum electromagnetic transmission signal.
▪ Product center averaging based data extraction: averaged TL 17235, RTL 17234 and PT 17236 values extracted from the registered measurement data profiles (the TL-profile 17216, the RTL-profile 17217 and the PT-profile 17218), where the averaged TL 17235, RTL 17234 and PT 17236 values are calculated from an averaging window centered around the product 13 center, corresponding to halfway its interaction time.

Next, the extracted TL, RTL and PT values are passed to an optional filtering software module 1724, were averaged TL, RTL and PT values over multiple accepted products are calculated and latched to the output register of the module.

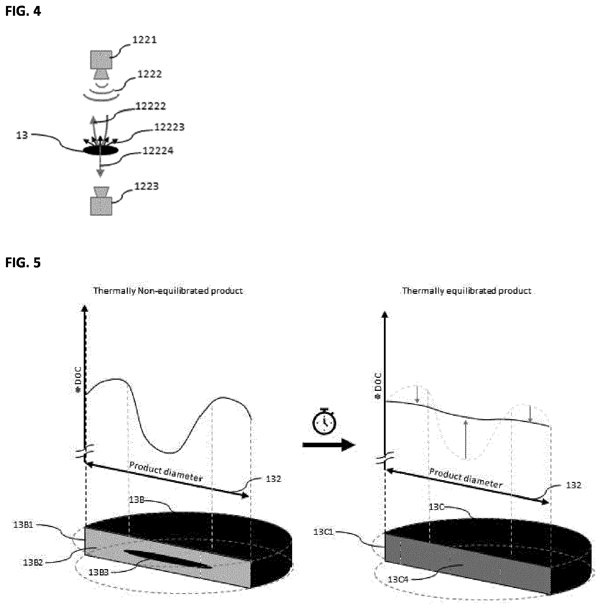

The method 1 comprises a software module 18 for calculating the normalized absorbance of the product 13 under test. This module takes as a input the PT, TL and RTL values from the static or dynamic mode branch depending on the selected mode-of-operation. First, the transmission loss is compensated for electromagnetic reflection 12222 and scattering 12223 losses (see also **FIG. 4**), resulting in a product specific absorbance. This product specific absorbance is then furthermore normalized for the product thickness to obtain a product specific absorbance per product thickness unit (e.g. expressed as dB/mm). Note that the electromagnetic reflection loss 12222 can be estimated from the product dielectric permittivity derived within software module 111. Alternatively the electromagnetic reflection loss 12222 could also be measured by implementing a transceiver functionality within the electromagnetic transmitter 1221.

The method 1 comprises a software module 19 for calculating the DOC based on the measured normalized product absorbance derived from software module 18. The interpolation table or analytical expression 113 generated by the software module 112 is then evaluated for the measured normalized product absorbance to obtain the DOC of the product under test 13.

**FIG. 5** illustrates the transition from a thermally non-equilibrated product 13B to a thermally equilibrated product 13C. **FIG. 5A** shows the cross-section 13B1 of a disk-like thermally non-equilibrated product 13B. A thermally non-equilibrated product 13B can be obtained after a fast cooling process (e.g. cryogenic freezing), whereby thermal energy is removed faster from the product surface than heat can propagate within the product, having only thermal diffusion as an internal driving force. Hence, short after a fast freezing process (prior to obtain thermal equilibrium within the product 13B), the product volume will consists of regions which are over frozen 13B2 and under frozen 13B3 compared to the final DOC value the product will have when it reaches thermal equilibrium.

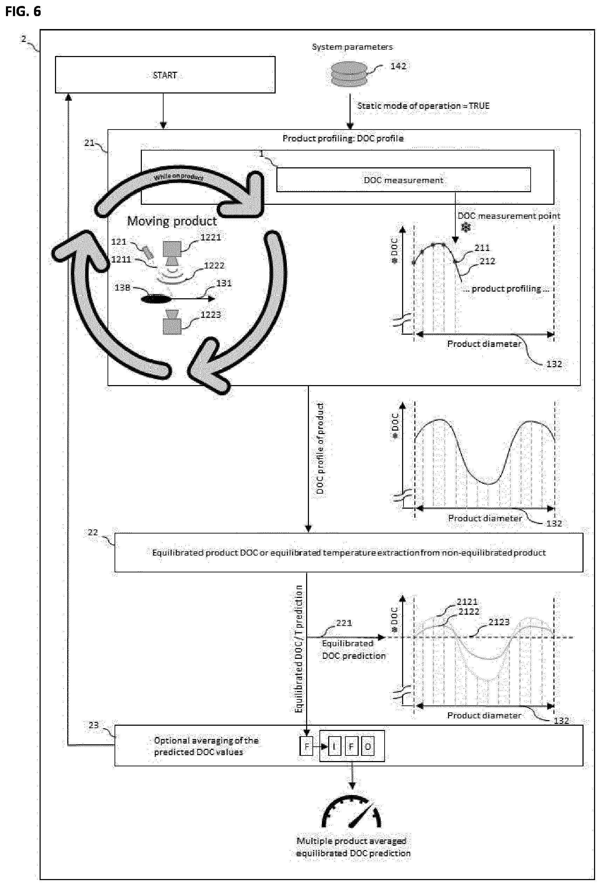

**FIG. 6** illustrates a method 2 for predicting the DOC of a thermally non-stabilized product 13B under test, passing the sensor 122.

The method 2 comprises a software module 21 for acquiring and calculating DOC values 211 on different positions of a thermally non-stabilized product 13B under test along the products 13B propagation path as it is passing the under the sensor 122. These DOC values 211 a stored in memory to form a product DOC profile 212.

The method 2, comprising a software module 22 for predicting the thermally equilibrated product DOC value 221 or the thermally equilibrated product temperature of the thermally non-stabilized product under test 13B, based on its DOC profile 212 and/or temperature profile. The software module 22 optionally comprising:
▪ Converting the product DOC profile to a product temperature profile
▪ Predicting the thermally equilibrated product temperature based on the said product temperature profile by considering the product as an insulated system (after the freezers outfeed / profile measurement) such that the law of energy conservation can be applied to the product and by assuming a constant product heat capacity within the temperature range under consideration. If the latter assumption is not met, one can first convert the said temperature profile to an enthalpy profile, taken into account the temperature dependent product heat capacity, from which the equilibrated temperature can be extracted.
   A potential implementation implies a convergence algorithm evaluates different equilibrated product temperatures (within the range of the temperature profile) such that the integrated product temperature profile value along the propagation axis becomes zero with respect to the equilibrated product temperature under test.
   Alternatively, one can use the averaged value calculated from the temperature profile as the predicted equilibrated product temperature.
▪ Converting the predicted equilibrated product temperature to a predicted equilibrated DOC value.

The method 2 optionally comprises a software module 23 for averaging the predicted equilibrated product DOC 221 and / or the predicted equilibrated product temperature, over multiple thermally non-stabilized products 13B.

## Claims

1. A method to calculate the degree-of-crystallization (further referred to as DOC) of a product or product layer under test (further referred to as 'the product" or "products") comprising:
▪ calculating the dielectric permittivity of the said product and its dependence on DOC,
▪ exposing the said product with electromagnetic radiation,
▪ acquiring the electromagnetic transmission and/or reflection signal,
▪ calculating the normalized electromagnetic transmission absorbance of the said product(s),
▪ calculating the DOC value taken into account the normalized electromagnetic transmission absorbance of the said product and the DOC dependence of the dielectric permittivity of the said product.

2. A method according to claim 1 wherein:
▪ the dielectric permittivity of the said product is calculated from the dielectric permittivities of the said product ingredients by means of an analytical mixing formula.

3. A method according to claim 2 wherein:
▪ the said product ingredients are given by a non-exhaustive and non-restrictive list of: freezable water fraction, non-freezable water faction, carbohydrate fraction, fat fraction, protein fraction, air fraction,...

4. A method according to any of the previous claims wherein:
▪ the exposed product surface, by the said electromagnetic radiation, is limited to a spot size significantly smaller than the said product lateral dimension.

5. A method according to any of the previous claims wherein:
▪ the electromagnetic radiation has a frequency within the range of 100 MHz to 1 THz.

6. A method according to any of the previous claims wherein:
▪ the said electromagnetic transmission signal consists of an electromagnetic power/intensity measurement or S-parameters obtained from a vector network analyser.

7. A method according to any of the previous claims comprising:
▪ calculating, defining or measuring the electromagnetic reflected signal, resulting in a reflection loss,
and wherein
▪ the said electromagnetic transmission absorbance is calculated from the electromagnetic transmission signal, compensated for the said reflection loss.

8. A method according to any of the previous claims comprising:
▪ calculating, defining or measuring the electromagnetic scattering loss,
and wherein
▪ the said electromagnetic transmission absorbance is calculated from the electromagnetic transmission signal, compensated for the said scattering loss.

9. A method according to any of the previous claims comprising:
▪ defining or measuring the product thickness,
and wherein
▪ the said electromagnetic transmission absorbance is normalized for the said product thickness.

10. A method according to any of the previous claims comprising:
▪ making an interpolation / lookup table for the dependence between the DOC and the normalized electromagnetic transmission absorbance,
wherein
▪ The said DOC value is interpolated on or searched within the said table.

11. A method according to claim 1-9 wherein
▪ the said DOC value is calculated from an analytical expression describing the relation between DOC value and the normalized electromagnetic transmission absorbance.

12. A method according any of the previous claims wherein
▪ the said product has not reached thermal equilibrium at the time of measurement,
▪ the said electromagnetic transmission signal is acquired at different positions on the said product,
▪ the said DOC value is calculated for multiple said positions, resulting in a DOC measurement profile for the said product,
and additionally comprising
▪ predicting the DOC value that the said product will have when thermal equilibrium is achieved based on the said DOC measurement profile of the said product.

13. A method according to claim 12 comprising:
▪ calculating an estimated temperature profile from the said DOC measurement profile,
▪ extracting the predicted equilibrium product temperature from the said temperature profile, and wherein
▪ the said DOC value that the said product will have when thermal equilibrium is achieved is calculated from the said predicted equilibrium product temperature.

14. A method according to claim 13 comprising:
▪ defining or measuring the freezing point of the said product,
and wherein:
▪ the conversions between DOC and temperature (T) are based on an analytical expression taken into account the said freezing point (T_{F}) of the said product: $DOC = 1 - \left({T}_{F}/T\right) .$

15. A method according to claims 12-14 comprising:
▪ calculating the temporal and / or spatial integration of:
∘ the said product DOC measurement profile and / or
∘ the said product temperature profile and / or
∘ any other said product signal profile, directly or indirectly deduced from the said the electromagnetic transmission signal,
further referred to as "the integrated profile signal",
wherein
▪ the said the integrated profile signal is used to calculate the said predicted DOC value.

16. A method according to any of the claims 12-15 comprising:
▪ averaging of the said predicted DOC values of the said products which were exposed to similar process conditions.
